# EUROPÄISCHE PATENTSCHRIFT

(11) **EP 2 338 565 B1**
(45) Veröffentlichungstag und Bekanntmachung des Hinweises auf die Patenterteilung: **16.09.2020**
(21) Anmeldenummer: 10192897.6
(22) Anmeldetag: 29.11.2010
(51) Int. Cl.: A61N 1/37, A61N 1/378

(54) **Geschaltete Schutzvorrichtung gegen elektromagnetische Störungen**
Switched protective device against electromagnetic interference
Dispositif de protection commuté contre les perturbations électromagnétiques

(30) Priorität: 22.12.2009 US 288857 P
(43) Veröffentlichungstag der Anmeldung: 29.06.2011
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Keck, Hans-Georg

(56) Entgegenhaltungen:
- EP-A1- 1 846 093
- EP-A2- 1 469 907
- US-A- 5 817 136
- US-A- 5 968 083
- US-B1- 7 561 915

## Beschreibung

Die Erfindung bezieht sich auf eine Vorrichtung und ein Verfahren zum Umgang mit elektromagnetischen Feldern, speziell solche Felder wie sie bei bildgebenden Kernspintomographie- (im folgenden MRT oder MRI) Geräten auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindikation kann durch ein aktives implantiertes medizinisches Gerät (im Folgenden auch Implantat oder IMD) gegeben sein. Neben den MRI-Untersuchungen stellen aber auch andere technische Anwendungen eine Gefahr für die Nutzer von medizinischen Geräten oder implantierbaren medizinischen Geräten dar, besonders wenn diese starke elektromagnetische Störfelder (EMI, Electro Magnetic Interference) in Ihrer Umgebung erzeugen.

Um MRI-Untersuchungen oder den Umgang im Umfeld von elektromagnetischen Störfeldern dennoch zu ermöglichen, sind verschiedene Ansätze, die sich entweder auf die Durchführung der MRI-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen. Diese technologischen Ansätze sind allerdings wenig spezifisch und erzeugen einen erhöhten Energiebedarf, was zu einer kürzeren Betriebsdauer bei gleichen Energiereserven führt.

Ein weiterer technologischer Ansatz ist das Verwenden von optischer Signalübertragung anstelle der typischen Elektrodenleitungen (205, 206, 207), die auf der elektrischen Signalübertragung beruhen. Durch die Verwendung dieser optischen Leitungen verhindert man das Einkoppeln der elektromagnetischen Störfelder eines MRI-Gerätes in die Elektrodenleitungen, aber das Gesamtsystem wird komplexer, da einerseits die elektrischen Signale erst in optische Signale umgewandelt werden müssen und aus den optischen Signalen am Stimulationsort an Hand der Signale die Stimulationsimpulse generiert werden müssen und andererseits am Stimulationsort gemessene Signale ebenfalles umgewandelt werden müssen. Solche komplexeren Systeme erhöhen in der Regel auch den Energiebedarf eines Implantats. Ein solches System, dass auf optischer Signalleitung basiert ist in der US 2005/0090886 und der US 7,450,996 beschrieben.

Auch ist aus der US 2002/0133204 und US 6,901,292 ein System bekannt, dass durch eine Auswahl von bestimmten Schutzvorrichtungen verschiedene Modi zum Betreiben eines elektrischen Implantates vorsieht. Nachteil dieses Systems ist, dass es weiterhin für hohe RF-Felder eine Erwärmung der Elektroden zulässt, da der Fokus der Schriften auf der elektromagnetischen Kompatibilität mit Metalldetektoren und elektronischen Sicherheitseinrichtungen liegt.

US 5,968,083 A beschreibt eine aktive Schaltung, um ein Gerät vor Überspannungstransienten zu schützen.

Aufgabe der Erfindung ist es, eine Vorrichtung und eine Methode für medizinische Geräte und implantierbare medizinische Geräte bereitzustellen, die die Nachteile des Stands der Technik beheben und einen sicheren Betrieb in Gegenwart von elektromagnetischen Störfeldern zu erlauben.

Die Aufgabe wird durch ein medizinisches Gerät (MD) mit den Merkmalen des Anspruchs 1 gelöst.

Dabei umfasst das medizinische Gerät (MD) eine Einheit zur Erkennung von elektromagnetischen Störungen (230) mit mindestens einem Sensor und/oder Indikator für elektromagnetische Störfelder und einer Schaltvorrichtung (240), mindestens einer Elektrode, mindestens einer Steuereinheit, die mit der Einheit zur Erkennung von elektromagnetischen Störungen verbunden oder verbindbar ist und mindestens einer Detektionseinheit und/oder mindestens einer Stimulationseinheit, die jeweils mit der mindestens einen Steuereinheit und der mindestens einen Elektrode verbunden oder verbindbar sind, wobei die mindestens eine Elektrode in Kontakt mit Körpergewebe ist und dass bei einer Erkennung von elektromagnetischen Störfeldern durch die Einheit zur Erkennung von elektromagnetischen Störungen die Eingangscharakteristik der mindestens einen Elektrode zur Elektronik des MD oder IMD mittels der mindestens einen Schaltvorrichtung (240) automatisch so verändert wird, dass die Einflüsse der elektromagnetischen Störfelder minimiert werden.

Beispielsweise wird die Eingangscharakteristik mit mindestens einem EMI-Kondensator und/oder einen Schwingkreis und/oder anderer geeigneter Bauteile verändert. Bei einem EMI-Kondensator handelt es sich um einen Kondensator zur Verringerung von Einflüssen von elektromagnetischer Störstrahlung. Die Einheit zur Erkennung von elektromagnetischen Störungen kann aus einem Sensor und/oder Indikator für elektromagnetische Felder und/oder einer Kombination aus mehreren Sensoren und/oder Indikatoren für elektromagnetische Felder bestehen. Beispiele für solche Sensoren und Indikatoren sind, ohne sich auf diese zu beschränken, GMR-Sensoren, MagFET-Sensoren, Hallsensoren, elektrooptische Wandler, die Überwachung von Batteriespannungen während Kondensatorladeprozessen, die Detektion von RF-Feldern, die Detektion von magnetischen Grandientenfeldern, die Detektion von durch elektromagnetische Felder induzierten Strömen und die Detektion von spezifischen Vibrationen oder als Sensoren ausgelegte Bauteile zur Detektion von durch Lorentzkräfte induzierten Vibrationen.

Bevorzugt wird, dass das MD ein implantierbares medizinisches Gerät (IMD) ist. Auch bevorzugt ist, dass die mindestens eine Elektrode in einer Gewebetasche unter der Haut und/oder in einer Herzkammer und/oder in einem Herzkranzgefäß, wie dem Coronar Sinus, befindet.

Besonders bevorzugt handelt es sich bei dem MD um einen externen Herzschrittmacher und/oder Defibrillator und/oder um einen implantierbaren Herzschrittmacher und/oder einen implantierbaren Cardioverter/Defibrillator (ICD) und/oder ein implantierbares CRT-Gerät (Cardiac Resynchronization Therapy-Gerät) und/oder einen Neurostimulator und/oder ein Gerät zur Überwachung von physiologischen Funktionen, wie, aber nicht beschränkt auf, ein Gerät zum Überwachen der Herzfunktion.

Bevorzugt wird auch, dass die Eingangscharakteristik des MD über eine Schaltvorrichtung (240) mit einem oder mehreren Schaltgliedern veränderbar ist.

Des Weiteren wird bevorzugt, dass die Schaltvorrichtung (240) über eine lineare oder nicht lineare Kennlinie verfügt, wobei die Kennlinieneigenschaften durch ein spezielles Bauteil und/oder Material realisiert werden. Ausgenutzt werden beispielsweise die Kennlinien eines spannungsgesteuerten Kondensators, eines spannungsgesteuerten Widerstandes (z. B. FET Transistor) oder einer Pindiode. Ebenso wird die Kennlinie des Materialkomposits genutzt, das einen GMR Sensor realisiert. Als weitere Baugruppe zu dem hier genannten Schaltzweck ist ein mehrachsig sensibler Reed Schalter, zum Beispiel, drei Reedschalter die orthogonal in drei Raumrichtungen ausgerichtet sind.

Auch ist bevorzugt, dass die Schaltvorrichtung (240) mit einem oder mehreren EMI-Kondensatoren (310) in Reihe geschaltet oder die Schaltvorrichtung (240) mit einer bestehenden EMI-Kondensatorschaltung parallel geschaltet ist.

Ebenfalls bevorzugt wird, dass die Eingangscharakteristik mittels Bauteilen mit spannungsabhängiger Kapazität veränderbar ist. Insbesondere können die EMI-Kondensatoren (311) selbst als spannungsgesteuerte Kondensatoren ausgeführt sein.

Bevorzugt wird auch, dass die Schaltvorrichtung (240) so ausgestaltet ist, dass die EMI-Kondensatoren (410) mit einer veränderbaren und/oder vorbestimmbaren und/oder automatisch an die gemessenen Feldstärkenparameter angepassten Frequenz umgepolt werden, wobei die Umpolungen nur bei bestimmten von der Einheit zur Erkennung von elektromagnetischen Störungen erkannten Feldparametern oder permanent vorgenommen werden.

Besonders bevorzugt wird, dass die Umschaltung mittels einer so genannten H-Schaltung (410) erfolgt.

Des Weiteren wird bevorzugt, dass die Umpolungsfrequenzen der einzelnen Schaltglieder der Schaltvorrichtung unterschiedlich und/oder zeitinvariant und/oder zeitvariant sind.

Auch ist bevorzugt, dass die Umpolungsfrequenz unter Berücksichtigung der Polarität der durch die Elektrodenleitung (205, 206, 207) geflossenen Ladung bestimmt wird.

Die Ladungsbestimmung kann beispielsweise auf dem Stromintegral beruhen und/oder sich aus einer Spannungsmessung der bekannten Kapazität und der messtechnisch ermittelten Elektrodenimpedanz, jeweils während gleicher Polarität, berechnen.

Ebenfalls bevorzugt wird, dass das Schaltglied der Schaltvorrichtung (240) ein veränderbarer Widerstand oder veränderbarer Kondensator oder ein Halbleiter oder eine Kombination dieser ist.

Besonders bevorzugt ist das Halbleiterschaltglied ein Transistor, Thyristor, Diac (Diode for Alternating Current), Triac (Triode for Alternating Current switch) und/oder eine Diode.

Bevorzugt wird auch, dass das Schalten der Schaltvorrichtung (240) oder die Veränderung des Arbeitspunktes auf der Kennlinie elektrisch, optisch, akustisch, magnetisch oder thermisch ausgelöst wird.

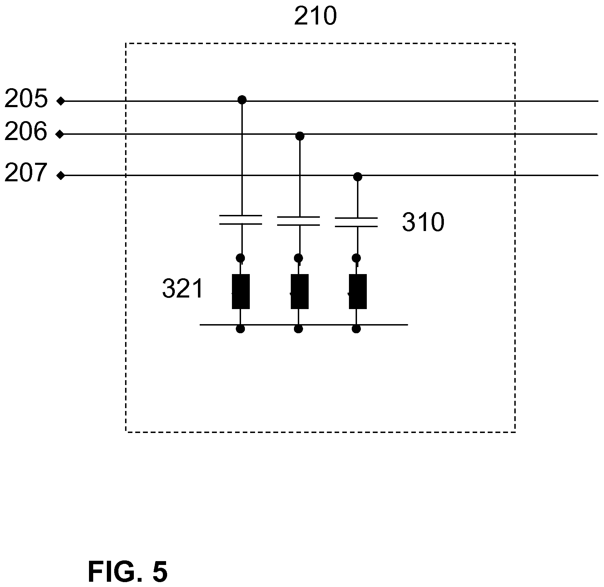

Außerdem bevorzugt ist in Sonderfällen die Ankopplung der Eingangsleitungen über die EMI Kondensatoren nur dann zu gewährleisten, wenn die Störung eine bestimmte Frequenz hat bzw. in einem bestimmten Frequenzbereich liegt. Die Schaltvorrichtung kann insofern als frequenzabhängiger Schalter betrachtet werden, d. h. als eine Vorrichtung deren Impedanz nur im Umfeld, wie aber nicht beschränkt auf eine Bandbreite < 10 MHz, einer bestimmten Frequenz um mehr als 3dB abnimmt. Dies kann durch eine LC Reihenschaltung realisiert werden, wie in Figur 5 beispielhaft gezeigt.

Auch wird bevorzugt, dass die Schaltvorrichtung frequenzabhängig funktioniert und als LC Reihenschaltung ausgeführt ist, wobei die Resonanz der LC Reihenschaltung auf die abzuleitende Störfrequenz einstellbar ist. Beispielsweise kann das Einstellen unter Ausnutzung der ohnehin vorhandenen EMI Kapazität vornehmbar sein. Die Resonanz ist auf die abzuleitende Störfrequenz einstellbar, und kann zum Beispiel durch programmierte Umschaltung der L und/oder C Komponenten eingestellt werden.

Des Weiteren wird bevorzugt, dass zusätzlich eine Kommunikationseinheit vorhanden ist, die ausgestaltet ist, eine unidirektionale oder bidirektionale Verbindung zu einem vorhandenen MRI-Gerät herzustellen.

Besonders bevorzugt wird die unidirektionale oder bidirektionale Verbindung mittels einer Telemetrie und/oder einer RF-Kommunikation und/oder auf Basis von RFID und/oder der Induzierung von gezielten elektromagnetischen Störungen hergestellt. Ebenfalls möglich sind auch Verbindungen basierend auf anderen nicht drahtgebundenen Verfahren, wie aber nicht beschränkt auf, Mobilfunkverfahren, Datenübertragungsstandards wie WLAN, Bluetooth und/oder Certified Wireless USB.

Ebenfalls besonders bevorzugt ist, dass das MRI-Gerät über die Kommunikationseinheit des MD oder IMD ein Schalten der Schaltvorrichtung (240) auszulösen und/oder zu steuern und/oder den Arbeitspunkt auf der Kennlinie der Schaltvorrichtung (240) zu verändern vermag.

Auch ist bevorzugt, dass die Einheit zur Erkennung von elektromagnetischen Störungen zur Detektion von elektromagnetischen Feldern mindestens einen der folgenden Sensoren oder Indikatoren umfasst, GMR-Sensor, MagFET-Sensor, Hallsensor, elektrooptische Wandler als Indikator, die Überwachung von Batteriespannungen während Kondensatorladeprozessen als Indikator, die Detektion von RF-Feldern als Indikator, die Detektion von magnetischen Grandientenfeldern als Indikator, die Detektion von durch elektromagnetische Felder induzierten Strömen als Indikator, die Detektion von spezifischen Vibrationen oder als Sensoren ausgelegte Bauteile zur Detektion von durch Lorentzkräfte induzierten Vibrationen als Indikator.

Des Weiteren ist bevorzugt, dass zusätzlich zur Änderung der Eingangscharakteristik mindestens eine der folgenden Maßnahmen bei der Detektion von elektromagnetischen Störfeldern eingeleitet wird: das Wechseln in einen MRI-sicheren Zustand, ein verlängertes Verbleiben in einem MRI-sicheren oder gegenüber elektromagnetischen Störfeldern unempfindlichen Zustand, eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima, und die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

Des Weiteren wird bevorzugt, dass ein Lagesensor zur Plausibilitätsprüfung herangezogen wird und eine positive MRI-Erkennung nur erfolgt, wenn der Lagesensor eine liegende Positur und/oder eine andere voreinstellbare Positur meldet.

Besonders bevorzugt ist der Lagesensor selbst kalibrierend, wobei die Kalibrierung unter voreinstellbaren Randbedingungen, wie, aber nicht beschränkt auf, Uhrzeiten und/oder Herzraten und/oder Atemfrequenz und/oder hämodynamische Parameter und/oder Aktivität (Bewegungssensor) stattfindet.

Bevorzugt wird auch ein Verfahren zur Herstellung eines MRI-sicheren Zustands eines MD unter Verwendung eines der MDs nach den Ansprüchen 1 bis 15.

Beschreibung der Figuren:

Einige Aspekte der Erfindung werden in den Figuren 1 -8 dargestellt.
- Fig. 1: schematische Darstellung des Ablaufs einer MRI-Untersuchung
- Fig. 2: Blockschaltbild einer erfindungsgemäßen geschalteten EMI-Schutzvorrichtung
- Fig. 3: Detailreicheres Blockschaltbild einer erfindungsgemäßen geschalteten EMI-Schutzvorrichtung
- Fig. 4: Blockschaltbild einer Variante einer erfindungsgemäßen geschalteten EMI-Schutzvorrichtung
- Fig. 5: Blockschaltbild einer Variante einer erfindungsgemäßen geschalteten EMI-Schutzvorrichtung
- Fig. 6: Blockschaltbild einer Variante einer erfindungsgemäßen geschalteten EMI-Schutzvorrichtung
- Fig. 7: Blockschaltbild einer Variante einer erfindungsgemäßen geschalteten EMI-Schutzvorrichtung mit steuerbaren Kondensatoren
- Fig. 8: Blockschaltbild einer Variante einer erfindungsgemäßen geschalteten EMI-Schutzvorrichtung mit einer H-Schaltung

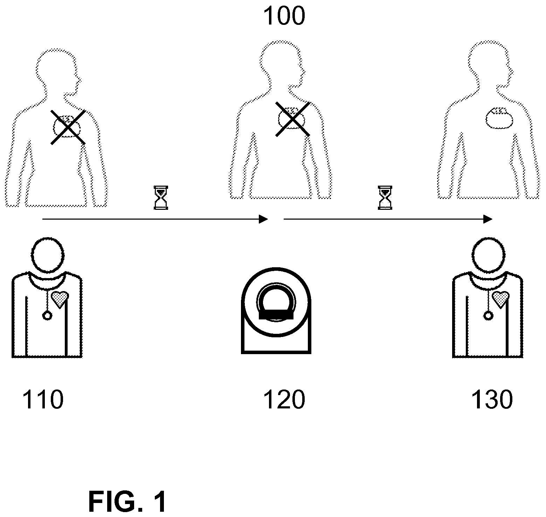

Figur 1 beschreibt den Stand der Technik, bei dem der ICD-Patient (100) vor der geplanten MRT-Untersuchung von einem Kardiologen nachgesorgt und der ICD ausgeschaltet (110) wird. Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen.

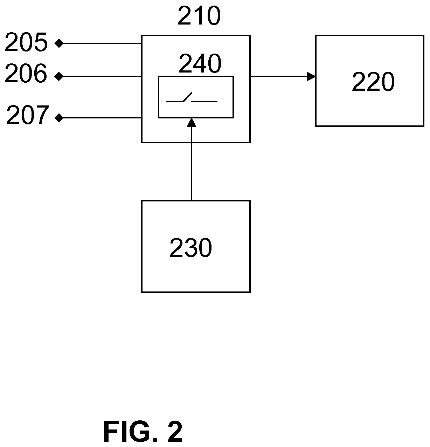

Figur 2 zeigt ein Blockschaltbild der erfindungsgemäßen Lösung, bei der die eingehenden Elektrodenleitungen (206 rechtsventrikulär, 205 rechtsatrial und 207 Schockleitung zur Abgabe von Hochenergieimpulsen) mit der Eingangsschutzschaltung (210) verbunden sind und die Charakteristik der Eingangsschutzschaltung (210) von einer Einheit zur Erkennung von elektromagnetischen Störungen (230) veränderbar ist. Wobei die Eingangscharakteristiken der Elektroden zur Elektronik des MD oder IMD mit der Schaltvorrichtung (240) zusammen als Eingangsschutzschaltung (210) bezeichnet werden. Die durch die Eingangsschutzschaltung (210) veränderbaren oder manipulierbaren Signale der Elektrodenleitungen (205, 206, 207) werden an die Implantatselektronik (220) weitergeleitet. Für die Erfindung ist es, wie für den Fachmann klar ersichtlich, unerheblich, ob das Implantat über eine oder mehrere Elektrodenleitungen (205, 206, 207) verfügt. Es sind auch Varianten vorstellbar, in denen eine, mehrere oder alle Elektroden nur als Flächen auf der Außenseite des Implantates ausgeführt sind.

Die Erfindung ermöglicht, dass Vorkehrungen für die erforderliche MRT Sicherheit oder allg. Sicherheit in starken RF-Feldern nur bei Vorhandensein dieser Randbedingung getroffen werden und in dem die weitaus überwiegende Zeit erforderlichen Funktionsmodus des Implantats die Einstellungen zugunsten der Signalqualität optimiert werden. Ferner berücksichtigt die erfindungsgemäße Lösung, dass zu hohe EMI-Kondensatoren (310) das Gefahrenpotential der Stimulation durch Gradientenfelder im MRT erhöhen.

Allgemein beschrieben heißt das, dass beim Erkennen eines starken elektromagnetischen Feldes, insbesondere RF-Felder wie im Umfeld von MRT Scannern auftretend, die ein Gefährdungspotential für Patient und Implantat darstellen, wird automatisch die EMI Schutzschaltung des Implantats umkonfiguriert. Im einfachsten Fall werden hierzu die EMI Kondensatorwerte erhöht oder überhaupt erst die EMI-Kondensatoren (310) zugeschaltet oder einer bestehenden Schutzschaltung parallel geschaltet. Deren gemeinsamer Kontaktpunkt z. B. am Implantatsgehäuse kann hierzu durch einen Schalter aufgetrennt werden, um im alltäglichen Betrieb des Implantats die hochauflösende und breitbandige Signalaufzeichnung insbesondere auch die Impedanzermittlung für einen hämodynamischen Sensor zu ermöglichen. Zur Minimierung der Wechselwirkungen mit RF-Feldern sind besonders hohe EMI Kondensatoren erforderlich. Dies erhöht jedoch andererseits das Risiko, dass Gradientenfelder stimulationsfähige Ströme induzieren können, die über die hohen EMI Kondensatoren einen Pfad geringer Impedanz finden würden. Erfindungsgemäß wird dieses Risiko minimiert, indem die Polarität der Kondensatoren repetitiv geändert wird, so dass während einer Polarität nicht ausreichend Ladung in eine Richtung fließen kann, um das Herzmuskelgewebe zu depolarisieren. Es wird also ermöglicht, die Anforderungen an eine hohe Störfestigkeit und eine sehr hohe Signalaufzeichnungsqualität in einem elektronischen Implantat zu kombinieren. Ebenso minimiert diese Lösung auch das Risiko einer ungewollten und damit potentiell arrhythmogenen Stimulation durch Gradientenfelder.

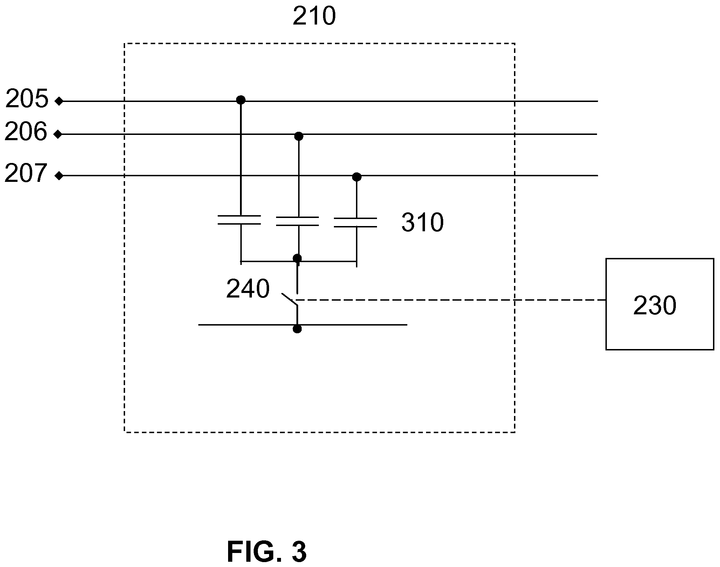

In einem anderen Ausführungsbeispiel, Figur 3, werden EMI-Kondensatoren (310) in Abhängigkeit von den Messungen der Einheit zur Erkennung von elektromagnetischen Störungen (230) vom Gehäuse durch eine Schaltvorrichtung (240) entkoppelt. Dabei sind die EMI-Kondensatoren (310) und die Schaltvorrichtung (240) Teil der Eingangsschutzvorrichtung und die EMI-Kondensatoren (310) werden über eine gemeinsame Schaltvorrichtung (240) geschaltet.

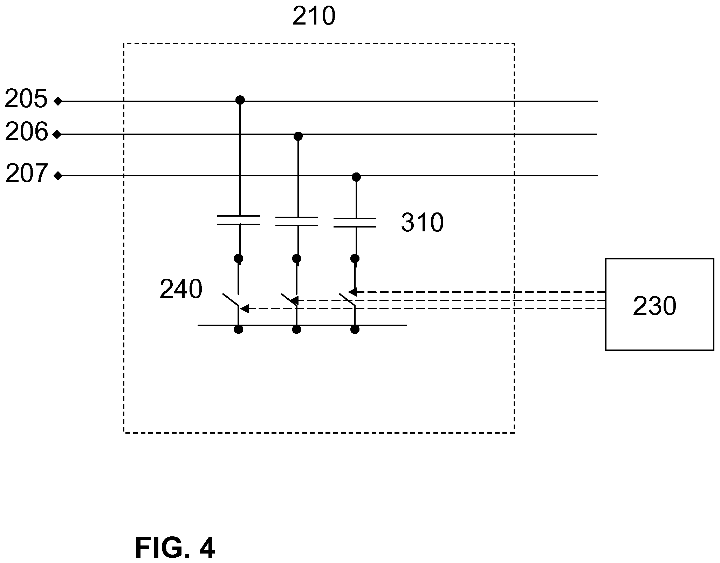

Die Ausführungsvariante in Figur 4 ist analog zu der aus Figur 3 aufgebaut, mit dem Unterschied, dass jeder EMI-Kondensator über eine eigene Schaltvorrichtung (240) verfügt, die jeweils separat von der Einheit zur Erkennung von elektromagnetischen Störungen (230) geschaltet werden können.

Es ist für die erfindungsgemäße Lösung in Sonderfällen ausreichend, die Ankopplung der Eingangsleitungen über die EMI Kondensatoren nur dann zu gewährleisten, wenn die Störung eine bestimmte Frequenz hat bzw. in einem bestimmten Frequenzbereich liegt. Die Schaltvorrichtung kann insofern als frequenzabhängiger Schalter betrachtet werden, d. h. als eine Vorrichtung deren Impedanz nur im Umfeld (z. B. Bandbreite < 10 MHz) einer bestimmten Frequenz um mehr als 3dB abnimmt. Dies kann durch eine LC Reihenschaltung realisiert werden, wie in Figur 5. Die Induktivität (321) ist auf den EMI Kondensator (320) so abgestimmt, das Resonanz bei der Frequenz (Abweichung im Rahmen der genannten Bandbreite) auftritt, für die die Eingangsleitung mit einer möglichst geringen Impedanz aus dem Implantatsgehäuse geschaltet werden soll. Die Verluste der Bauelemente L und C sind bei deren Auswahl so zu berücksichtigen, dass die Güte des Schwingkreises Q>10 beträgt.

Um diese Lösung innerhalb desgleichen Implantats für verschiedene Störfrequenzen anbieten zu können, sieht die erfindungsgemäße Lösung vor, dass die C oder L (oder beide) umschaltbar sind. Die Umschaltung kann in Abhängigkeit der Störfelderkennungseinheit geschehen (diese stellt die Frequenz der Störung fest und wählt die dazu passende LC Kombination). Die Umschaltung kann alternativ durch das externe Programmiergerät vorgenommen werden.

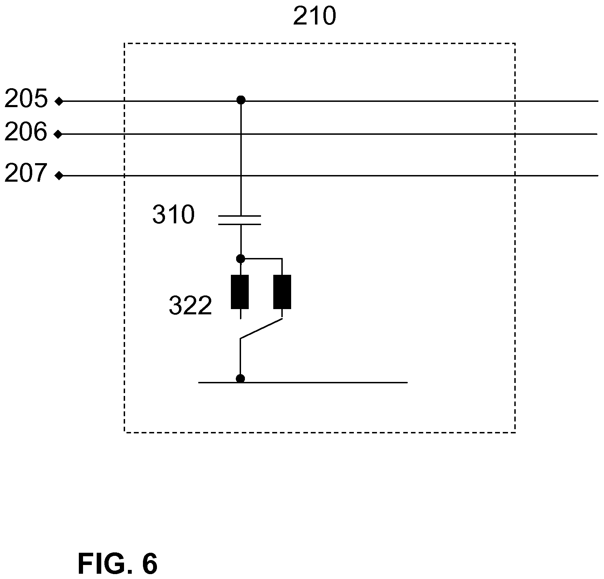

Die Figur 6 zeigt nur das Beispiel der Umschaltbaren Induktivität (322). Um die Güte einzustellen (verringern), kann die Induktivität über einen entsprechenden Widerstand geshuntet werden. Die Vorrichtung ist so dimensioniert (L, C inkl. deren Verluste, und ggf. dem Shunt Widerstand), dass bei Resonanz die Eingangsleitungen dadurch mit einer Impedanz < 200 Ohm (pro Leitung) an das Gehäuse gekoppelt sind.

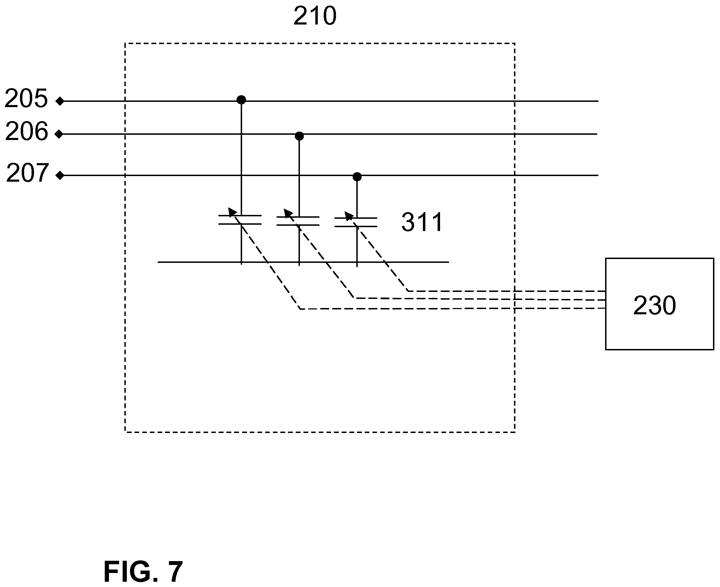

Die Figur 7 zeigt ein Ausführungsbeispiel, bei dem anstelle der EMI-Kondensatoren (310) steuerbare EMI-Kondensatoren (311) verwendet werden, die jeweils direkt von der Einheit zur Erkennung von elektromagnetischen Störungen (230) gesteuert werden und daher auf eine Schaltvorrichtung (240) verzichten können. Bei der in der Figur 7 dargestellten Schaltung handelt es sich um eine so genannte H-Schaltung, wie sie bevorzugt zur Umpolung verwendet werden kann.

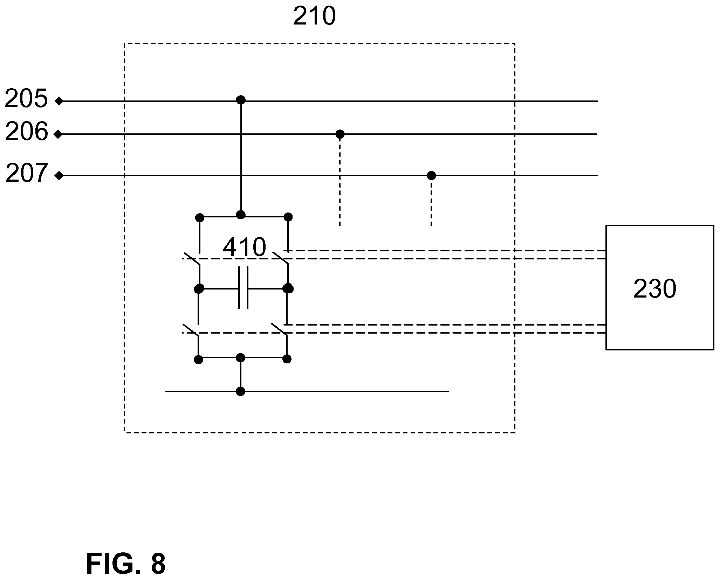

Figur 8 zeigte eine Eingangsschutzschaltung (210) analog zu der in Figur 3, wobei der geschaltete EMI-Kondensator (410) durch eine H-Schaltung geschaltet wird und so periodisch umgepolt werden kann. Die H-Schaltung für den geschalteten EMI-Kondensator (410) wird durch die Einheit zur Erkennung von elektromagnetischen Störungen (230) gesteuert. In Figur 8 ist nur ein geschalteter EMI-Kondensator (410) in einer H-Schaltung dargestellt, die gestrichelten Linien symbolisieren einen analogen Aufbau für die anderen Elektrodenleitungen (206 und 207). In einer Variante dieses Ausführungsbeispiels werden alle Elektrodenleitungen (205, 206 und 207) mit einem EMI-Kondensator (410) in einer H-Schaltung verbunden.

## Patentansprüche

1. Medizinisches Gerät (MD) mit einer Einheit zur Erkennung von elektromagnetischen Störungen (230) mit:
- mindestens einem Sensor und/oder Indikator für elektromagnetische Störfelder und einer Schaltvorrichtung (240),
- mindestens einer Elektrode,
- mindestens einer Steuereinheit, die mit der Einheit zur Erkennung von elektromagnetischen Störungen verbunden oder verbindbar ist und
- mindestens einer Detektionseinheit und/oder mindestens einer Stimulationseinheit, die jeweils mit der mindestens einen Steuereinheit und der mindestens einen Elektrode verbunden oder verbindbar sind,
ein oder mehrere EMI-Kondensatoren, oder eine EMI-Kondensatorschaltung,
wobei das Gerät derart adaptiert ist dass wenn die mindestens eine Elektrode in Kontakt mit Körpergewebe ist und bei einer Erkennung von elektromagnetischen Störfeldern durch die Einheit zur Erkennung von elektromagnetischen Störungen die Eingangscharakteristik der mindestens einen Elektrode zur Elektronik des MD oder IMD mittels der mindestens einen Schaltvorrichtung (240) automatisch so verändert wird, dass die Einflüsse der elektromagnetischen Störfelder minimiert werden, **gekennzeichnet durch** Bauteile mit spannungsabhängiger Kapazität, wobei die Schaltvorrichtung (240) mit besagten einem oder mehreren EMI-Kondensatoren (310) in Reihe geschaltet oder die Schaltvorrichtung (240) mit besagtem bestehenden EMI-Kondensatorschaltung parallel geschaltet ist, wobei das Gerät derart ausgebildet ist, dass die Eingangscharakteristik mittels besagten Bauteilen mit spannungsabhängiger Kapazität veränderbar ist.

2. MD nach einem der vorangegangenen Ansprüche, **dadurch gekennzeichnet, dass** das MD ein implantierbares medizinisches Gerät (IMD) ist.

3. MD nach einem der vorangegangenen Ansprüche, **dadurch gekennzeichnet, dass** die Schaltvorrichtung (240) über eine lineare oder nicht lineare Kennlinie verfügt, wobei die Kennlinieneigenschaften durch ein spezielles Bauteil oder Material realisiert werden.

4. MD nach einem der vorangegangenen Ansprüche, **dadurch gekennzeichnet, dass** die Schaltvorrichtung (240) so ausgestaltet ist, dass die EMI-Kondensatoren (410) mit einer veränderbaren und/oder vorbestimmbaren und/oder automatisch an die gemessenen Feldstärkenparameter angepassten Frequenz umgepolt werden, wobei die Umpolungen nur bei bestimmten von der Einheit zur Erkennung von elektromagnetischen Störungen erkannten Feldparametern oder permanent vorgenommen werden.

5. MD nach Anspruch 4, **dadurch gekennzeichnet, dass** die Umpolungsfrequenzen von einzelnen Schaltgliedern der Schaltvorrichtung unterschiedlich und/oder zeitinvariant und/oder zeitvariant sind.

6. MD nach einem der Ansprüche 4 oder 5, **dadurch gekennzeichnet, dass** die Umpolungsfrequenz unter Berücksichtigung der Polarität der durch die Elektrodenleitung (205, 206, 207) geflossenen Ladung bestimmt wird.

7. MD nach einem der vorangegangenen Ansprüche, **dadurch gekennzeichnet, dass** das Schaltglied der Schaltvorrichtung (240) ein veränderbarer Widerstand oder veränderbarer Kondensator oder ein Halbleiter oder eine Kombination dieser ist.

8. MD nach einem der vorangegangenen Ansprüche, **dadurch gekennzeichnet, dass** das Schalten der Schaltvorrichtung (240) oder die Veränderung des Arbeitspunktes auf der Kennlinie elektrisch, optisch, akustisch, magnetisch oder thermisch ausgelöst wird.

9. MD nach einem der vorangegangenen Ansprüche, **dadurch gekennzeichnet, dass** die Schaltvorrichtung frequenzabhängig funktioniert und als LC Reihenschaltung ausgeführt ist, wobei die Resonanz der LC Reihenschaltung auf die abzuleitende Störfrequenz einstellbar ist.

10. MD nach einem der vorangegangenen Ansprüche, **dadurch gekennzeichnet, dass** zusätzlich eine Kommunikationseinheit vorhanden ist, die ausgestaltet ist, eine unidirektionale oder bidirektionale Verbindung zu einem vorhandenen MRI-Gerät herzustellen.

11. MD nach Anspruch 10, **dadurch gekennzeichnet, dass** das MRI-Gerät über die Kommunikationseinheit des MD oder IMD ein Schalten der Schaltvorrichtung (240) auszulösen und/oder zu steuern und/oder den Arbeitspunkt auf der Kennlinie der Schaltvorrichtung (240) zu verändern vermag.

12. MD nach einem der vorangegangenen Ansprüche, **dadurch gekennzeichnet, dass** die Einheit zur Erkennung von elektromagnetischen Störungen zur Detektion von elektromagnetischen Feldern mindestens einen der folgenden Sensoren oder Indikatoren umfasst:
- GMR-Sensor,
- MagFET-Sensor,
- Hall sensor,
- elektrooptische Wandler als Indikator,
- die Überwachung von Batteriespannungen während Kondensatorladeprozessen als Indikator,
- die Detektion von RF-Feldern als Indikator,
- die Detektion von magnetischen Grandientenfeldern als Indikator,
- die Detektion von durch elektromagnetische Felder induzierten Strömen als Indikator,
- die Detektion von spezifischen Vibrationen oder als Sensoren ausgelegte Bauteile zur Detektion von durch Lorentzkräfte induzierten Vibrationen als Indikator.

13. MD nach einem der vorangegangenen Ansprüche, **dadurch gekennzeichnet, dass** zusätzlich zur Änderung der Eingangscharakteristik mindestens eine der folgenden Maßnahmen bei der Detektion von elektromagnetischen Störfeldern eingeleitet wird:
- das Wechseln in einen MRI-sicheren Zustand
- ein verlängertes Verbleiben in einem MRI-sicheren oder gegenüber elektromagnetischen Störfeldern unempfindlichen Zustand,
- eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima, und
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit, neben der Störung auch Informationen auf diese Weise zu übertragen und auf dem Bildschirm des MRI sichtbar zu machen.

## Claims

1. A medical device (MD) comprising a unit for detecting electromagnetic interference (230), having:
- at least one sensor and/or indicator for electromagnetic interference fields and a switching device (240);
- at least one electrode;
- at least one control unit, which is connected or connectable to the unit for detecting electromagnetic interference; and
- at least one detection unit and/or at least one stimulation unit, which each are connected or connectable to the at least one control unit and the at least one electrode,
- one or more EMI capacitors, or an EMI capacitor circuit,
the device being adapted in such a way that, when the at least one electrode is in contact with bodily tissue and if electromagnetic interference fields are detected by the unit for detecting electromagnetic interference, the input characteristic of the at least one electrode for the electronics system of the MD or IMD is automatically modified by means of the at least one switching device (240) in such a way that the influences of the electromagnetic interference fields are minimised,
**characterised by** components having voltage-dependent capacitance,
the switching device (240) being connected in series with said one or more EMI capacitors (310) or the switching device (240) being connected in parallel with said existing EMI capacitor circuit,
the device being designed in such a way that the input characteristic is modifiable by means of said components having voltage-dependent capacitance.

2. The MD according to the preceding claim, **characterised in that** the MD is an implantable medical device (IMD).

3. The MD according to either one of the preceding claims, **characterised in that** the switching device (240) has a linear or non-linear characteristic curve, the characteristic curve properties being realised by a specific component or material.

4. The MD according to any one of the preceding claims, **characterised in that** the switching device (240) is designed such that the polarity of the EMI capacitors (410) is reversed using a frequency that may be modified and/or may be predetermined and/or is automatically matched to the measured field-intensity parameters, the polarity reversals being performed only with specific field parameters detected by the unit for detecting electromagnetic interference, or being performed permanently.

5. The MD according to claim 4, **characterised in that** the polarity reversal frequencies of individual switching elements of the switching device are different and/or time-invariant and/or time-variant.

6. The MD according to either one of claims 4 or 5, **characterised in that** the polarity reversal frequency is determined taking into account the polarity of the charge flowing through the electrode lead (205, 206, 207).

7. The MD according to any one of the preceding claims, **characterised in that** the switching element of the switching device (240) is a modifiable resistor or modifiable capacitor or a semiconductor or a combination thereof.

8. The MD according to any one of the preceding claims, **characterised in that** the switching of the switching device (240) or the modification of the operating point on the characteristic curve is triggered electrically, optically, acoustically, magnetically, or thermally.

9. The MD according to any one of the preceding claims, **characterised in that** the switching device functions frequency-dependently and is configured as an LC series circuit, wherein the resonance of the LC series circuit may be set to the interference frequency to be diverted.

10. The MD according to any one of the preceding claims, **characterised in that** a communication unit is additionally provided and is configured to establish a unidirectional or bidirectional connection to an MRI device that is present.

11. The MD according to claim 10, **characterised in that** the MRI device is able to trigger and/or control switching of the switching device (240) and/or modify the operating point on the characteristic curve of the switching device (240) by means of the communication unit of the MD or IMD.

12. The MD according to any one of the preceding claims, **characterised in that** the unit for detecting electromagnetic interference for the detection of electromagnetic fields comprises at least one of the following sensors or indicators:
- GMR sensor,
- MagFET sensor,
- Hall sensor,
- electro-optical converter as indicator,
- monitoring of battery voltages during capacitor charging processes as indicator,
- detection of RF fields as indicator,
- detection of magnetic gradient fields as indicator,
- detection of currents induced by electromagnetic fields as indicator,
- detection of specific vibrations or components designed as sensors for the detection of vibrations induced by Lorentz forces as indicator.

13. The MD according to any one of the preceding claims, **characterised in that**, in addition to modifying the input characteristic, at least one of the following measures is initiated in the event that electromagnetic interference fields are detected:
- a change is made to an MRI safe state,
- an MRI safe state or a state that is insensitive to electromagnetic interference fields is maintained for an extended period of time,
- electrical measurements (for example impedance measurements) are synchronised using field intensity minimum values occurring with periodic or pulsed electromagnetic fields, or a stimulation is synchronised using these same minimum values, and
- electromagnetic pulses are emitted for signalling that a medical device, especially an implant, is present in the electromagnetic field, especially for signalling to an MRI device, with the possibility of thus transmitting information in addition to the interference and visualising same on the screen of the MRI device.

## Revendications

1. Appareil médical (MD) doté d'une unité permettant la détection de perturbations électromagnétiques (230) avec :
- au moins un capteur, et/ou un indicateur, de champs de perturbations électromagnétiques et un dispositif de commutation (240),
- au moins une électrode,
- au moins une unité de commande, qui est reliée ou peut être reliée avec l'unité de détection de perturbations électromagnétiques et
- au moins une unité de détection, et/ou au moins une unité de stimulation, qui sont respectivement reliées ou peuvent être reliées avec l'au moins une unité de commande et l'au moins une électrode,
un ou plusieurs condensateurs d'interférence électromagnétique EMI, ou un circuit de condensateurs EMI,
où l'appareil est adapté de telle manière que si
l'au moins une électrode est en contact avec du tissu corporel et, lors d'une détection de champs de perturbation électromagnétique par l'unité permettant la détection de perturbations électromagnétiques, la caractéristique d'entrée de l'au moins une électrode est changée de manière automatique pour l'électronique de l'appareil MD ou de l'appareil implantable IMD au moyen du dispositif de commutation (240) de telle manière que les effets des champs de perturbation électromagnétiques soient minimisés,
**caractérisé par** des composants dotés de capacités variables avec la tension,
où le dispositif de commutation (240) est branché en série avec ledit un condensateur ou lesdits condensateurs EMI (310), ou le dispositif de commutation (240) est branché en parallèle avec un dit circuit de condensateurs EMI en place,
où l'appareil est conçu de manière à ce que la caractéristique d'entrée puisse être modifiée au moyen de dits composants avec une capacité variable avec la tension.

2. Appareil médical selon l'une des revendications précédentes, **caractérisé en ce que** le MD est un appareil médical implantable (IMD).

3. Appareil médical selon l'une des revendications précédentes, **caractérisé en ce que** le circuit de commutation (240) dispose d'une courbe caractéristique linéaire ou non linéaire, où les propriétés de la courbe caractéristique sont concrétisées par un composant ou un matériau spécial.

4. Appareil médical selon l'une des revendications précédentes, **caractérisé en ce que** le dispositif de commutation (240) est conçu de telle manière que les condensateurs EMI (410) sont repolarisés avec une fréquence variable, et/ou pouvant être prédéfinie, et/ou ajustée automatiquement aux paramètres d'intensités de champs mesurées, où les repolarisations ne peuvent être effectuées que pour des paramètres de champ déterminés par l'unité pour la détection de perturbations électromagnétiques reconnus.

5. Appareil médical selon la revendication 4, **caractérisé en ce que** les fréquences de repolarisation d'éléments de commutation individuels du dispositif de commutation sont divers, et/ou invariables dans le temps, et/ou variables dans le temps.

6. Appareil médical selon l'une des revendications 4 ou 5, **caractérisé en ce que** la fréquence de repolarisation est déterminée en tenant compte de la polarité de la charge circulant à travers la ligne d'électrode (205, 206, 207).

7. Appareil médical selon l'une des revendications précédentes, **caractérisé en ce que** l'élément de commutation du dispositif de commutation (240) est une résistance variable, ou un condensateur variable, ou un semi-conducteur, ou une combinaison de ceux-ci.

8. Appareil médical selon l'une des revendications précédentes, **caractérisé en ce que** la commutation du dispositif de commutation (240) ou la modification du point de travail sur la courbe caractéristique est déclenchée électriquement, optiquement, acoustiquement, magnétiquement ou thermiquement.

9. Appareil médical selon l'une des revendications précédentes, **caractérisé en ce que** le dispositif de commutation fonctionne de manière dépendante avec la fréquence et est conçu sous forme de circuit en série à courant faible LC, où la résonance du circuit en série LC peut être réglée sur la fréquence de perturbation à évacuer.

10. Appareil médical selon l'une des revendications précédentes, **caractérisé en ce qu'**en supplément une unité de communication est présente, qui est conçue pour établir une connexion unidirectionnelle ou bidirectionnelle vers un appareil d'imagerie par résonance magnétique IRM

11. Appareil médical selon la revendication 10, **caractérisé en ce que** l'appareil d'IRM peut déclencher une commutation du dispositif de commutation (240), et/ou commander, et/ou modifier le point de travail sur la courbe caractéristique du dispositif de commutation (240) par le biais de l'unité de communication de l'appareil médical ou de l'appareil médical implantable.

12. Appareil médical selon l'une des revendications précédentes, **caractérisé en ce que** l'unité de détection de perturbations électromagnétiques permettant la détection de champs électromagnétiques comprend au moins un des capteurs ou indicateurs suivants :
- un capteur à magnétorésistance géante GMR,
- un capteur à transistor à effet de champ magnétique MagFET,
- un capteur à effet Hall,
- un convertisseur électro-optique servant d'indicateur,
- le suivi de tensions de batterie pendant des processus de charge de condensateur servant d'indicateur,
- la détection de champs de radiofréquences RF servant d'indicateur,
- la détection de champs à gradients magnétiques servant d'indicateur,
- la détection de flux induits par des champs électromagnétiques servant d'indicateur,
- la détection de vibrations spécifiques ou de composants conçus sous la forme de capteurs pour la détection de vibrations induites par des forces de Lorentz servant d'indicateur.

13. Appareil médical selon l'une des revendications précédentes, **caractérisé en ce qu'**en outre, pour la modification de la caractéristique d'entrée, au moins une des mesures suivantes est mise en œuvre lors de la détection de champs de perturbation électromagnétiques :
- le changement vers un état d'IRM sûr,
- un séjour prolongé dan un état d'IRM sûr ou un état insensible vis-à-vis de champs de perturbations électromagnétiques,
- une synchronisation de mesures électriques (par exemple, de mesures d'impédance) avec des minimas d'amplitudes d'intensités de champs se produisant pour des champs électromagnétiques périodiques ou pulsés ou la synchronisation d'une stimulation avec ces minimas d'amplitude en question, et
- l'émission d'impulsions électromagnétiques pour la signalisation qu'un appareil médical, spécialement un implant, est présent dans le champ électromagnétique, spécialement pour la signalisation à un appareil d'IRM, avec la possibilité, à côté de la perturbation, de transmettre également des informations de cette manière et à les rendre visibles sur l'écran de l'IRM
